(19) Europäisches Patentamt
European Patent Office
Office européen des brevets

(11) EP 1 311 248 B1

(12) EUROPÄISCHE PATENTSCHRIFT

(45) Veröffentlichungstag und Bekanntmachung des Hinweises auf die Patenterteilung:
**02.05.2007 Patentblatt 2007/18**

(21) Anmeldenummer: **01978305.9**

(22) Anmeldetag: **24.08.2001**

(51) Int Cl.:
***A61K 9/70*** *(2006.01)* ***A61K 31/48*** *(2006.01)*
***A61P 25/14*** *(2006.01)* ***A61P 5/08*** *(2006.01)*
***A61P 5/24*** *(2006.01)*

(86) Internationale Anmeldenummer:
**PCT/EP2001/009823**

(87) Internationale Veröffentlichungsnummer:
**WO 2002/015889 (28.02.2002 Gazette 2002/09)**

(54) **TRANSDERMALES THERAPEUTISCHES SYSTEM ZUR BEHANDLUNG DES RESTLESS-LEGS-SYNDROMS**

TRANSDERMAL THERAPEUTIC SYSTEM FOR TREATING RESTLESS-LEGS-SYNDROME

SYSTEME THERAPEUTIQUE TRANSDERMIQUE POUR LE TRAITEMENT DU SYNDROME DES JAMBES SANS REPOS

(84) Benannte Vertragsstaaten:
**AT BE CH CY DE DK ES FI FR GB GR IE IT LI LU MC NL PT SE TR**

(30) Priorität: **24.08.2000 DE 10043321**

(43) Veröffentlichungstag der Anmeldung:
**21.05.2003 Patentblatt 2003/21**

(73) Patentinhaber: **Axxonis Pharma AG**
**10963 Berlin (DE)**

(72) Erfinder:
- **HOROWSKI, Reinhard**
**14129 Berlin (DE)**
- **TACK, Johannes**
**13595 Berlin (DE)**
- **ENGFER, Adalbert**
**12309 Berlin (DE)**

(74) Vertreter: **Wablat, Wolfgang**
**Patentanwalt**
**Potsdamer Chaussee 48**
**14129 Berlin (DE)**

(56) Entgegenhaltungen:
**EP-A- 0 137 278**
**EP-A- 1 027 889**
**WO-A-99/48484**
**DE-A- 4 116 912**
**DE-A- 19 938 823**
**US-A- 5 229 129**
**US-A- 5 399 355**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 311 248 B1

## Beschreibung

Die Erfindung betrifft eine Verwendung eines transdermalen therapeutischen Systems (TTS) aufweisend eine Arzneimittelschicht, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat oder dessen Salz enthält zur Herstellung eines Arzneimittels zur Behandlung des Restless-Legs-Syndroms.

Das Restless-Legs-Syndrom (RLS) ist eine neurologische Erkrankung, die in allen Altersstufen, jedoch gehäuft im höheren Lebensalter, auftritt und deren Beschwerden sich hauptsächlich dadurch äußern, dass infolge von Dysästhesien und Parästhesien Krämpfe und Schmerzen in den Beinen auftreten, die einen Drang zur Bewegung auslösen. Da diese Symptome meist nachts oder in Phasen verminderter Aktivität wie beim Sitzen und Ausruhen sich bemerkbar machen, führt der Bewegungsdrang tagsüber zu Ruhelosigkeit und nachts zu Schlafstörungen. Dies beeinträchtigt die Lebensqualität der Betroffenen erheblich.

Es ist bekannt, dass die Behandlung des Restless-Legs-Syndroms mit oralen Einmalgaben dopaminerger Medikamente wie Lisurid am Abend zu einer Verbesserung der Beschwerden führt und die allgemeine Beeinträchtigung der Lebensqualität positiv beeinflusst wird. Im Gegensatz zur Parkinson-Therapie, bei der dopaminerge Pharmaka und ihre Kombinationen über den Tag verteilt eingenommen werden, ist bei der einmaligen peroralen Einnahme zur Behandlung des Restless-Legs-Syndroms die Entwicklung einer Toleranz gegen die akuten (anflutungsbedingten) dopaminergen Nebenwirkungen beeinträchtigt, d.h. mit jeder wirksamen Dosis können die bekannten Nebenwirkungen wie Orthostase, Hypotonie, Schwindel, Übelkeit und Erbrechen auftreten. Auch unvorhersehbare und unkontrollierbare Schlafattacken, wie sie in letzter Zeit vermehrt berichtet werden, können auftreten. Weiterhin ist die Plasmakonzentration nicht konstant, sondern großen Schwankungen unterworfen, und zwar nicht nur aus kinetischen Gründen, sondern auch abhängig von den individuellen Randbedingungen der Einnahme (Art und Zeitpunkt der Nahrungsaufnahme, etc.). Daher besteht auch die Gefahr einer zeitweisen Überdosierung mit der Folge von beispielsweise einer REM-Unterdrückung und daraus resultierenden Problemen und Schlafstörungen.

Außerdem sind bei den peroralen dopaminergen Therapien auch Rebound-Phänomene am Folgetag sowie sogenannte Augmentationen, d.h. erhöhter Tonus, Unruhe und Bewegungsdrang, häufig.

Der Erfindung liegt das technische Problem zugrunde, ein Mittel zur Behandlung des Restless-Legs-Syndroms anzugeben, welches nebenwirkungsfrei ist, zumindest jedoch gegenüber der oralen Gabe deutlich reduzierte Nebenwirkungen zeigt, insbesondere den Wirkstoff langsam anfluten lässt und in Höhe und Dauer gut steuerbar ist.

Hier kann ein transdermales therapeutisches System gemäß der nachfolgend beschriebenen Erfindung eine auch individuell gewünschte kontrollierte Wirkdauer (ggf. durch Entfernen des Pflasters) erreichen. Gegenüber der peroralen Gabe wird mit dem TTS die Bioverfügbarkeit erhöht, was dazu führt, dass die Gesamtdosis, die zum Erreichen der therapeutisch gewünschten Wirkung erforderlich ist, reduziert werden kann. Die $\alpha$-adrenolytische Wirkung von Lisurid und seinen Derivaten hat bei dieser Form der Anwendung den weiteren Vorzug, dass auch nächtlicher Harndrang und andere Blasen-Funktionsstörungen, wie er bei Restless-Legs- und auch Parkinson-Patienten nicht selten ist (u. a. auch durch Prostata-Hyperplasie), deutlich gebessert wird, was gleichfalls zum Therapieerfolg beiträgt.

Die Erfindung betrifft die Verwendung eines transdermalen therapeutischen Systems (TTS) aufweisend eine Arzneimittelschicht, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat gemäß Formel I oder dessen physiologisch verträgliches Salz mit einer Säure enthält,

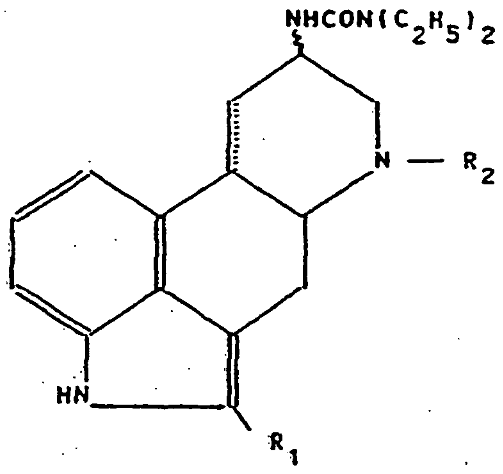

Formel I

worin ------ eine Einfachbindung oder eine Doppelbindung ist, worin R1 ein H-Atom oder ein Halogenatom, insbesondere ein Bromatom, ist, und worin R2 C1-4-Alkyl, insbesondere Methyl, ist, als Mittel zur Behandlung des Restless-Legs-Syndroms.

Mit der Erfindung wird als besonderer Vorteil erreicht, dass - im Gegensatz zur üblichen oralen Einmalgabe pro Tag - eine kontinuierlicher Wirkstofflux eingerichtet wird und somit die Plasmakonzentrationen sich definiert einstellen und hinsichtlich des Verlaufes kontrollieren lassen. Dies verhindert weitgehend die bei oralen Einmalgaben zu beobachtenden dopaminergen Nebenwirkungen, wie Müdigkeit, Schwindel, Erbrechen, Obstipation u.a. Denn es hat sich herausgestellt, dass diese Nebenwirkungen sich vermeiden lassen, wenn die Plasma-Konzentrationen des Wirkstoffes nicht großen und schnellen Schwankungen unterworfen werden, wie bei oraler Gabe sich automatisch einstellend, sondern langsam und kontinuierlich eingestellt werden. Hinzu kommt, dass die Problematik oraler Gaben, wie stark veränderliche Absorptionsgeschwindigkeiten und wenig definierter Zeitpunkt der Maximalkonzentration im Plasma, beispielsweise abhängig von Art und Zeitpunkt der Nahrungsaufnahme, mit der Erfindung praktisch eliminiert werden. Insbesondere eine Überdosierung (und folglich REM-Unterdrückung sowie andere Schlafmusterstörungen) wird vermieden. Weiterhin kann vergleichsweise schnell abgesetzt werden, nämlich einfach durch Entfernung des TTS. Im Gegensatz zum Absetzen eines oral verabreichten Wirkstoffs erfolgt der Abbau im Plasma zügig und kontrolliert, wodurch auch "hang over", Rebound oder Augmentationen vermieden werden können. Schließlich ist eine individuelle Dosierung durch Auswahl des Flux F und/oder der wirksamen Fläche unschwer möglich. Vorzugsweise werden F und wirksame Fläche so ausgewählt, dass sich eine Dosis im Bereich von 10 $\mu$g bis 2 mg Wirkstoff, vorzugsweise 50 bis 200 $\mu$g (beispielsweise Lisurid), pro Tag einstellt.

Bevorzugt ist es, wenn die Matrix und/oder die Diffusionsbarriere ausgewählt ist mit der Maßgabe, dass der transdermale Flux F durch Humanhaut, gemessen gemäß Beispiel 1, im Bereich von 0,1 bis 2,0 $\mu g/cm^2/h$ liegt.

Als Ergolinderivate kommen beispielsweise in Frage: Bromlisurid (3-(2-Brom-9,10-didehydro-6-methyl-8$\alpha$-ergolinyl)-1,1-diethylharnstoff), Tergurid (3-(6-methyl-8$\alpha$-ergolinyl)-1,1-diethylharnstoff) und Protergurid (3-(6-propyl-8$\alpha$-ergolinyl)-1,1-diethylharnstoff). Bevorzugt ist es allerdings, wenn das Ergolin-Derivat Lisurid (3-(9,1-0-didehydro-6-methyl-8$\alpha$-ergolinyl)-1,1-diethylharnstoff) oder dessen physiologisch verträgliches Salz mit einer Säure ist. Die Herstellung von Lisurid und den weiteren erfindungsgemäß geeigneten Ergolinen wird beispielsweise in US 3,953,454, EP 056 358 und US 4,379,790 beschrieben. In Frage kommende Salze des Ergolin-Derivats sind beispielsweise Sulfate, Phosphate, Maleate, Citrate und Succinate sowie insbesondere Hydrogenmaleat.

Der Begriff des TTS umfasst insbesondere perkutan wirkende, aber auch transmucosal wirkende Systeme. Ein TTS ist typischerweise von flächiger Struktur und wird beispielsweise auf der Haut flächig zum Anliegen gebracht. Die Befestigung auf der Haut kann durch ein ggf. zusätzliches hautseitiges (und für den Wirkstoff permeables) Adhäsiv erfolgen. Ebenso kann die Matrix und/oder die Diffusionsbarriere selbst mit adhäsiven Eigenschaften ausgestattet sein. Schließlich kann ein nicht adhäsives TTS mittels weiterer Hilfsmittel, beispielsweise Klebebänder oder Bandagen, auf der Haut zum Anliegen gebracht werden. Als Matrix ist ein Stoff bezeichnet, in welchem der Wirkstoff immobilisiert ist. Demgegenüber ist der Wirkstoff in einem Wirkstoffreservoir nicht notwendigerweise immobilisiert, weswegen.das Wirkstoffreservoir ummantelt sein muss. Der hautseitige Teil des Mantels wird dabei von der Diffusionsbarriere gebildet. Es

versteht sich, dass der weitere Teil des Mantels möglichst inpermeabel, auch bezüglich Diffusionspfade, für den Wirkstoff sein sollte. Der Begriff immobilisiert meint in diesen Zusammenhängen, dass kein unkontrollierter Wirkstoff-Fluss möglich ist. Insbesondere Diffusion eines Wirkstoffes in einer Matrix und/oder durch eine Diffusionsbarriere ist jedoch nicht nur möglich, sondern gezielt eingerichtet.. Die Diffusionskoeffizienten bestimmen dabei letztendlich den Flux des Wirkstoffes aus dem TTS in die Haut eines Patienten. Die an die Haut eines Patienten abgegebene Dosis ist daher eine in erster Näherung lineare Funktion der wirksamen Fläche des TTS. Die wirksame Fläche ist die Kontaktfläche von für Wirkstoffe diffusionsoffenen Bereichen des TTS. TTS sind sowohl für humane als auch für veterinärmedizinische Zwecke einsetzbar.

Ein TTS des eingangs genannten Aufbaus ist grundsätzlich bekannt aus der Literaturstelle WO 92/20339. Hierin ist insbesondere der Effekt von Propylenglycol-Laurinsäure auf den Flux beschrieben, wodurch eine beachtliche Fluxerhöhung erreicht wird. Die angegebenen Werte beziehen sich dabei auf auf Hautproben aufgetragene Lösungen und nicht auf die eigentlichen TTS. Zu diesen sind keinerlei Angabe hinsichtlich des Flux gemacht. In einem TTS werden gegenüber Werten aus einem Lösungsauftrag erheblich niedrigere Fluxwerte erreicht.

Ein Lisurid enthaltendes TTS ist weiterhin bekannt aus der Literaturstelle WO 91/00746. Die darin angegebenen Flux-Werte für menschliche Hautproben sind nicht ohne weiteres auf erzielbare in-vivo-Werte übertragbar.

TTS des beschriebenen Aufbaus werden für verschiedene Indikationen, u.a. Parkinson, verwendet. Im Fall einer Behandlung der Parkinsonschen Krankheit sind möglichst hohe Dosen wünschenswert. Ein transdermales therapeutisches System verbessert hier zusätzlich die Compliance, die für die Kombinationstherapien dieser Krankheit und ihren meist alten und multimorbiden Patienten von sehr erheblicher Bedeutung ist. Eine bessere Steuerbarkeit sowie die Möglichkeit, circadiane Profile zu erreichen (z. B. mit geringer, möglichst konstanter Stimulation während der Nacht bzw. einer Pause) sind hier besonders günstig und bisher nicht erreicht (z. B. zur Vermeidung von Psychosen sowie zur Verbesserung der Schlafqualität). Im Falle der Ergolin-Derivate Lisurid, Tergurid und Bromergurid trägt auch deren dopaminpartialagonistische bzw. partialantagonistische Wirkung dazu bei, das Entstehen von Psychosen zu verhindern bzw. vorhandene Psychosen und ähnliche Probleme zu bessern.

Das TTS kann im einzelnen wie folgt ausgebildet sein. Auf der hautabgewandten Seite der Matrix und/oder des Wirkstoffreservoirs kann eine Deckschicht angeordnet sein. Diese kann beispielsweise mit Folien aus Polyethylen oder Polyester gebildet sein. Die Dicke beträgt typischerweise 10 bis 100 $\mu$m. Es ist möglich, zur Erzielung eines ausreichenden Lichtschutzes, die Deckschicht zu pigmentieren und/oder zu metallisieren. Als Metallisierung ist das Aufbringen einer sehr dünnen Schicht (typischerweise weniger als 1 $\mu$m, meist im 10-100 nm Bereich) eines Metalls, beispielsweise Aluminium, auf die Deckschicht bezeichnet. Pigmente können alle im Rahmen der Überzugsmittel gebräuchlichen Pigmente, auch Effektpigmente, sein, sofern sie physiologisch unbedenklich sind. Auf der Applikationsseite kann ein abziehbarer Liner vorgesehen sein, beispielsweise eine silikonisierte oder fluorpolymerbeschichtete polymere Schutzfolie.

Die Matrix und/oder Diffusionsbarriere kann einen Stoff, ausgewählt aus der Gruppe bestehend aus "Polyacrylat, Polyurethan, Celluloseether, Silikon, Polyvinylverbindungen, Silikat und Mischungen dieser Stoffe sowie Copolymere dieser Polymerverbindungen", vorzugsweise Polyacrylat, als Hauptmatrixkomponente aufweisen. Eine Hauptmatrixkomponente bildet zumindest 50 Gew.-%, beispielsweise zumindest 80-90 Gew.-% der Matrix (der Begriff der Matrix bezieht sich dabei auf die fertige Schicht, i.e. Hauptmatrixkomponente(n) mit Hilfsstoff(en) und Wirkstoff(en)). Eine Einstellung des gewünschten Flux erfolgt einerseits durch Auswahl des Stoffes in Abhängigkeit des Diffusionskoeffizienten des wirkstoffes darin und andererseits und ggf. in Abstimmung hiermit durch Wahl der Schichtdicke der Matrix in Richtung orthogonal zur Hautoberfläche. Der Dickenbereich einer Matrix liegt typischerweise im Bereich von 10 $\mu$m bis 500 $\mu$m.

Ein besonders bevorzugter Polyacrylatkleber als Hauptmatrixkomponente ist käuflich unter der Bezeichnung GELVA® multipolymer solution 7881, erhältlich von der Firma Monsanto Deutschland GmbH, Düsseldorf. Dabei wird ausdrücklich Bezug genommen auf das unter dieser Bezeichnung vertriebene Produkt gemäß Datenblatt in der Fassung vom 23.04.1996. Ebenfalls gut verwendbar ist Eudragit® E100, erhältlich von der Firma Röhm, Deutschland.

Mit den vorstehenden Polyacrylatklebern wird eine besonders vorteilhafte nichttriviale Eigenschaftskombination erhalten, nämlich optimaler Flux, gute Haftfähigkeit, gute Hautverträglichkeit und gute Haltbarkeit.

Die Diffusionsbarriere kann alternativ ein Polymer ausgewählt aus der Gruppe bestehend aus "Celluloseester, Celluloseether, Silikon, Polyolefin und Mischungen sowie Copolymere dieser Stoffe" als Hauptbarrierenkomponente aufweisen. Zum Begriff der Hauptbarrierenkomponente gilt das Vorstehende zur Hauptmatrixkomponente analog. Die Diffusionsbarriere kann als Folie mit einer Dicke von 10 $\mu$m bis 300 $\mu$m ausgebildet sein, wobei die Dicke der Schicht (in Verbindung mit dem Diffusionskoeffizienten des Wirkstoffes in dem Polymer) nach Maßgabe des gewünschten Flux eingestellt wird.

In der Matrix und/oder dem Wirkstoffreservoir und/oder der Diffusionbarriere können für TTS übliche Hilfsstoffe enthalten sein. Bevorzugterweise wird als Hilfsstoff ein penetrationsverstärkendes Mittel eingesetzt, welches vorzugsweise ausgewählt ist aus der Gruppe bestehend aus "C1-C8 aliphatische, cycloaliphatische und aromatische Alkohole, gesättigte und ungesättigte C8-18-Fettalkohole, gesättigte und ungesättigte C8-18 Fettsäuren, Kohlenwasserstoffe und Kohlenwasserstoffmischungen, Fettsäureester aus C3-19-Fettsäuren und C1-6-Alkylmonoolen, Dicarbonsäurediester

aus C4-8-Dicarbonsäuren und C1-6-Alkyl-monoolen, und Mischungen dieser Stoffe. Penetrationsverstärkende Mittel verbessern den Flux des Wirkstoffes durch die Haut, auf welche das TTS aufgebracht ist. Beispiele aus den vorgenannten Stoffen sind: 1,2-Propandiol, Menthol, Dexpanthenol, Benzylalkohol, Laurylalkohol, Isocetylalkohol, Cetylalkohol, Mineralöl, Laurinsäure, Isopalmitinsäure, Isostearinsäure, Ölsäure; Methylester, Ethylester, 2-Hydroxyethylester, Glycerolester, Propylester, Isopropylester, Butylester, sec.-Butylester oder Isobutylester der Laurinsäure, Myristinsäure, Stearinsäure oder Palmitinsäure. Bevorzugt ist der Einsatz von Dimethylisosorbid, Isopropylmyristat und Laurylalkohol, höchstbevorzugt von Laurylalkohol. Als weitere Hilfsstoffe kommen beispielsweise Kristallisationsinhibitoren in Frage. Als Kristallisationsinhibitoren sind hochdisperses Siliciumdioxid oder makromolekulare Stoffe wie Polyvinylpyrrolidone, Polyvinylalkohole, Dextrine, Dextrane, Sterine, Gallensäuren und insbesondere Vinylpyrrolidon-Vinylacetat-Copolymere geeignet wie Kollidon® VA 64.

Es versteht sich, dass jedenfalls das penetrationsverstärkende Mittel ebenfalls ausreichend durch die Matrix bzw. die Diffusionsbarriere diffundieren muss. Im Falle des Einsatzes einer Matrix sowie des Hilfsstoffes Laurylalkohol bildet der Laurylalkohol vorzugsweise 10 bis 30 Gew.-%, höchstvorzugsweise 15 bis 20 Gew.-%, der Matrix.

Die Hilfsstoffe können grundsätzlich 0 bis 50 Gew.-% der Matrix bilden. Der Wirkstoff kann 0,2 bis 20 Gew.-%, vorzugsweise 1 bis 10 Gew.-%, der Matrix bilden. Die Summe der Anteile an Matrixhauptkomponente, Hilfsstoffen und Wirkstoffen bildet dabei stets 100 Gew.-%.

Die Dosis des Wirkstoffes in einem das TTS tragenden menschlichen Körper hängt neben den vorstehenden diffusionsbezogenen Eigenschaften des TTS auch von dessen wirksamer Fläche mit der Haut ab. Wirksame Fläche meint hierbei die Fläche, mit welcher die Matrix oder die Diffusionsbarriere an der Haut anzuliegen kommt. Vorzugsweise erfolgt die Variation nach Maßgabe der gewünschten Dosis in einem Bereich von 1 bis 100 $cm^2$.

Im Rahmen der Erfindung lassen sich dabei, bei abgestimmten Flux für eine vorgegebene Indikation, leicht patientenindividuelle Dosisvariationen von einem Arzt einrichten, nämlich durch Wahl einer geeigneten Größe. Somit kann die Behandlung beispielsweise auf unterschiedliche Körpergewichte, Altersgruppen etc. unschwer abgestellt werden. Insbesondere ist es möglich, ein TTS, welches eine (eher große) Standardfläche aufweist, mit Unterteilungsmarkierungen für Teildosen auszustatten, so dass ein Anwender lediglich einen einer bestimmten Dosis entsprechenden Teilabschnitt abtrennen und anwenden kann. Entsprechende Aufdrucke lassen sich unschwer auf der Deckschicht anbringen.

Eine weitere Einsatzmöglichkeit ist die Verwendung eines erfindungsgemäßen TTS zur Herstellung eines Mittels zur Behandlung oder Prävention des prämenstruellen Syndroms oder seiner Symptome, wobei F vorzugsweise von 0,1 bis 0,5 $\mu g/cm^2/h$ beträgt, sowie die Verwendung zur Herstellung eines Mittels zur Lactationshemmung, wobei F vorzugsweise von 0,1 bis 0,5 $\mu g/cm^2/h$ beträgt.

Im Folgenden wird die Erfindung anhand von Beispielen näher erläutert.

Beispiel 1: Flux-Messung

Zur Flux-Messung wird eine FRANZ Durchfluss Diffusionszelle verwendet. Die Messfläche beträgt 2 $cm^2$. Als Hautprobe werden 4 $cm^2$ ventrale und dorsale Haut einer männlichen haarlosen Maus (MF1 hr/hr Ola/Hsd, erhältlich von Harlan Olac, UK) verwendet, wobei subkutanes Fettgewebe sorgfältig entfernt wird. Auf die eingesetzte Haut ist ein 2 $cm^2$ TTS appliziert. Gegenüberliegend ist das Akzeptormedium angeordnet. Es ist verdünntes HHBSS (Hepes Hanks Balanced Salt Solution) enthaltend 5,96 g/l Hepes, 0,35 g/l $NaHCO_3$ und 0,1 ml/l 10x HBSS (erhältlich von Gibco, Eggenstein, DE). Weiterhin sind 1000 I.E./ml Penicillin (Benzylpenicillin Kaliumsalt, erhältlich von Fluka, Neu-Ulm, DE).

Die Messung erfolgt im einzelnen wie folgt. Das zu messende TTS wird zunächst auf die Haut appliziert. Sofort danach wird die Haut in die Diffusionszelle montiert. Das Akzeptor Medium wird in Intervallen von 2h zwischen t=0 und t=6 h und von 8 h zwischen t=6 h und t=54h beprobt. Pro Stunde werden 1ml Akzeptormedium durch die Diffusionszelle mittels einer peristaltischen Pumpe gepumpt. Die Temperatur des Akzeptormediums wird mittels eines zirkulierenden Wasserbades kontrolliert und hält die Oberfläche der Haut auf einer Temperatur von 31 °C mit 1 °C Genauigkeit.

Die Wirkstoffkonzentration in dem Akzeptormedium wird gemäß folgender Details mittels eines Radioimmunoassays bestimmt.

Kalibrierungskurven: Diese werden unter Verwendung von zwei unterschiedlichen Methanollösungen von nicht radioaktivem Lisuridhydrogenmaleatsalz, enthaltend je 1 mg/ml, konstruiert. Diese Lösungen werden unterschiedlich mit BSA-Puffer (0,041 M $Na_2HPO_2*2H_2O$, 0,026 M $KH_2PO_4$, 0,154 M NaCl, 0,015 M $NaN_3$, 0,1% (w/v) BSA, pH 7, supplementiert mit 0,05% (w/v) Ascorbinsäure) verdünnt, um Lisurid free base Konzentrationen im Bereich von 1000 - 3,9 pg/0,1ml zu erhalten. Zusätzlich wird eine wirkstofffreie Probe (0pg) eingesetzt. Die Kalibrierungsproben werden dreifach analysiert. Die Lisurid-Konzentrationen werden mittels der pharmacokinetic RIO PC Software, 2.5, berechnet (andere übliche Software ist ebenfalls einsetzbar).

Probenpräparation: Vor der Analyse wird das Akzeptormedium mit BSA-Puffer verdünnt zwecks Einstellung von Konzentrationen im auswertbaren Bereich der Kalibrierungskurve. 100 µl verdünnte Probe werden direkt der radioimmunologischen Analyse unterzogen.

Antiserum: Das Antiserum (Kaninchen) ist erhältlich durch Immunisierung mit dem Immunogen Lisurid-1-succinyl-BSA. Die Verdünnung des Antiserums im Assay ist 1:12500.
Tracer: $^3$H-Lisuridhydrogenmaleat mit einer spezifischen Aktivität von 4,3 GBq/mg wird verwendet.
Inkubation: zu 0,7 ml BSA-Puffer werden 0,1 ml BSA-Puffer mit Wirkstoff, 0,1 ml Tracerlösung (ca. 5000 cpm/ 0,1 ml BSA-Puffer) und 0,1 ml verdünntes Antiserum (1:12500) gegeben und es wird für 18 h bei 4°C inkubiert.
Separierung: antikörpergebundenes Lisurid wird von freiem durch Zugabe von 0,2 ml Holzkohlesuspension (1,25%(w/v) und 0,125% (w/v) Dextran in BSA-Puffer) und Inkubation für 30 min. bei 0 °C getrennt. Die Holzkohle wird durch Zentrifugation bei 3000 g für 15 min. sedimentiert. Der Überstand (enthaltend antikörpergebundenen Wirkstoff) wird dekantiert und der radiometrischen Analyse zugeführt.
Radiometrische Analyse: Zum Überstand werden 4ml des Szintillations Cocktails Atomlight (NEN) gegeben. Die Zählung erfolgt mit einem WALLAC 1409 oder 1410 β-Szintillationszähler ohne quench control.
Auswertung: Der perkutane Haut Flux wird wie folgt berechnet:

$$F = (C * R) / (A * T),$$

wobei F den percutanen Flux [ng/cm$^2$/h], C die Wirkstoffkonzentration im Akzeptormedium [ng/ml], R den Akzeptormediumsfluss [1ml/h], A die Messfläche [2cm$^2$] und T das Beprobungszeitintervall [h] sind.
Maximaler transdermaler Wirkstoffflux wird direkt von den Daten genommen. Mittlere perkutane Fluxwerte werden während Tag 1 und Tag 2 des Experiments bestimmt, basierend auf der kumulativ absorbierten Dosis in dem Zeitintervall t=0-22 und t=22-54.
Angaben für die Herstellung von TTS

Beispiel 2: TTS A

15 mg Kollidon VA 64 (Kristallisationsinhibitor) werden in 15 mg Isopropanol gelöst. Dann werden 5 mg Lisurid eingestreut. 80 mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 μm Rakel auf einem silikonisierten Liner ausgezogen. Dann wird bei 60 °C für 20 min. getrocknet und schließlich eine Deckschicht auflaminiert.
Messungen des Flux gemäß Beispiel 1 ergeben für F einen Tag 1 Wert von 0,43, einen Tag 2 Wert von 0,44 und einen maximalen F von 0,85 (jeweils in μg/cm$^2$/h).

Beispiel 3: TTS B

12,5 mg Dimethylisosorbid werden mit 2 mg Lisurid in 15 mg Isopropanol suspendiert. 80 mg Polyacrylatkleber (Gelva 7881) werden in einem Becherglas vorgelegt und die vorstehende Suspension, unter Nachspülen mit 30 mg Isopropanol, hinzugegeben. Nach gründlicher Durchmischung wird das erhaltene kristallfreie Wetmix mit 500 μm Rakel auf einem silikonisierten Liner ausgezogen. Dann wird bei 60 °C für 20 min. getrocknet und schließlich eine Deckschicht auflaminiert.
Messungen des Flux gemäß Beispiel 1 ergeben für F einen Tag 1 Wert von 0,23, einen Tag 2 Wert von 0,28 und einen maximalen F von 0,50 (jeweils in μg/cm$^2$/h).

Beispiel 4: TTS C

27,2 mg Kollidon VA 64 (Kristallisationsinhibitor) und 16,3 mg Laurylalkohol werden bei 60 °C gelöst. Dann werden 2 mg Lisurid in dieser Lösung bei 60 °C gelöst. 39,38 mg Eudragit E100, 13,41 mg Citroflex 4A und 1,71 mg Bernsteinsäure werden bei 150-200 °C geschmolzen. Nach Abkühlung auf 80 °C wird die Lisuridlösung unter Rühren hinzugegeben. Bei 80 °C wird mit 500 μm Rakel auf einem silikonisierten Liner ausgezogen. Dann wird auf 20 °C abgekühlt und schließlich ggf. eine Deckschicht auflaminiert.
Messungen des Flux gemäß Beispiel 1 ergeben für F einen Tag 1 Wert von 0,90, einen Tag 2 Wert von 1,76 und einen maximalen F von 2,53 (jeweils in μg/cm$^2$/h).

**Patentansprüche**

**1.** Verwendung eines transdermalen therapeutischen Systems (TTS) aufweisend eine Arzneimittelschicht, welche zumindest eine einen Wirkstoff enthaltende Matrix und/oder ein Wirkstoffreservoir und hautseitig des Wirkstoffreservoirs eine wirkstoffpermeable Diffusionsbarriere sowie als Wirkstoff ein Ergolin-Derivat gemäß Formel I oder dessen physiologisch verträgliches Salz mit einer Säure enthält,

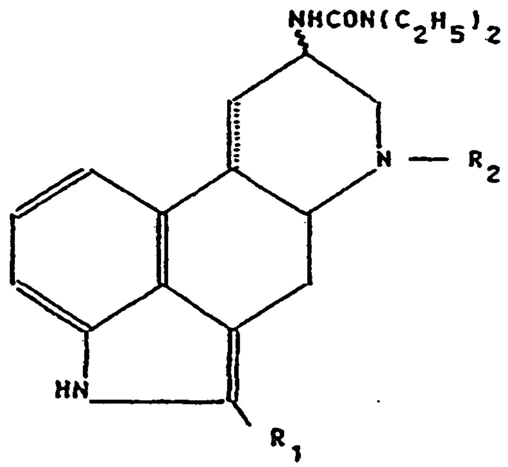

Formel I

worin ------- eine Einfachbindung oder eine Doppelbindung ist, worin R1 ein H-Atom oder ein Halogenatom, insbesondere ein Bromatom ist, und worin R2 C1-4-Alkyl ist,
zur Herstellung eines Mittels zur Behandlung des Restless-Legs-Syndroms.

**2.** Verwendung nach Anspruch 1, wobei die Matrix und/oder die Diffusionsbarriere ausgewählt ist mit der Maßgabe, dass der transdermale Fluss F durch Humanhaut, gemessen gemäß Beispiel 1, im Bereich von 0,1 bis 2,0 $\mu g/cm^2/h$ liegt.

**3.** Verwendung nach Anspruch 1 oder 2, wobei das Ergolin-Derivat Lisurid oder dessen Salz mit einer physiologisch verträglichen Säure ist.

**4.** Verwendung nach einem der Ansprüche 1 bis 3, wobei auf der hautabgewandten Seite der Matrix und/oder des Wirkstoffreservoirs eine Deckschicht angeordnet ist.

**5.** Verwendung nach einem der Ansprüche 1 bis 4, wobei die Matrix und/oder Diffusionsbarriere einen Stoff, ausgewählt aus der Gruppe bestehend aus "Polyacrylat, Polyurethan, Celluloseether, Silikon, Polyvinylverbindungen, Silikat und Mischungen dieser Stoffe sowie Copolymere dieser Polymerverbindungen", vorzugsweise Polyacrylat, als Hauptmatrixkomponente aufweist.

**6.** Verwendung nach einem der Ansprüche 1 bis 5, wobei die Diffusionsbarriere ein synthetisches Polymer ausgewählt aus der Gruppe bestehend aus "Celluloseester, Celluloseether, Silikon, Polyolefin und Mischungen sowie Copolymere dieser Stoffe" als Hauptbarrierenkomponente aufweist.

**7.** Verwendung nach einem der Ansprüche 1 bis 6, wobei die Matrix und/oder das Wirkstoffreservoir und/oder die Diffusionsbarriere ein penetrationsverstärkendes Mittel enthält, welches vorzugsweise ausgewählt ist aus der Gruppe bestehend aus "C1-C8 aliphatische, cycloaliphatische und aromatische Alkohole, gesättigte und ungesättigte C8-18-Fettalkohole, gesättigte und ungesättigte C8-18 Fettsäuren, Kohlenwasserstoffe und Kohlenwasserstoffmischungen, Fettsäureester aus C3-19-Fettsäuren und C1-6-Alkylmonoolen, Dicarbonsäurediester aus C4-8-Dicarbonsäuren und C1-6-Alkylmonoolen, und Mischungen dieser Stoffe.

**8.** Verwendung eines TTS nach einem der Ansprüche 1 bis 7 zur Herstellung eines Mittels zur Behandlung oder

Prävention des prämenstruellen Syndroms bzw. seiner Symptome, wobei F vorzugsweise von 0,1 bis 0,5 μg/cm²/h beträgt.

**9.** Verwendung eines TTS nach einem der Ansprüche 1 bis 7 zur Herstellung eines Mittels zur Lactationshemmung , wobei F vorzugsweise von 0,1 bis 0,5 μg/cm²/h beträgt.

**Claims**

**1.** Use of a transdermal therapeutic system (TTS) comprising a pharmaceutical layer containing at least one matrix having an active ingredient and/or an active ingredient reservoir and a diffusion barrier which is permeable to active ingredients and which is arranged on the skin side of the active ingredient reservoir and an ergoline derivative according to formula I or physiologically compatible salt thereof with an acid as active ingredient,

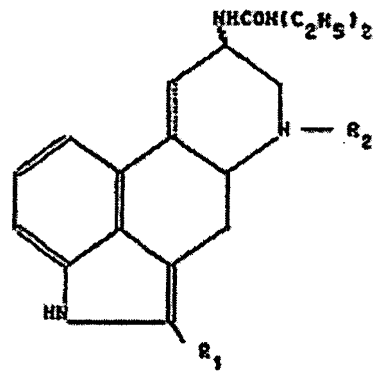

Formula I

wherein ------ is a single or double bond wherein R1 is an H atom or a halogen atom, particularly a bromine atom, and wherein R2 is C1-C4 alkyl
for the preparation of an agent for treating restless leg syndrome.

**2.** The use according to claim 1 wherein the matrix and/or diffusion barrier are selected so that the transdermal flux F through human skin measured as described in Example 1 is in the range from 0,1 to 2,0 μg/cm²/h.

**3.** The use according to claim 1 or 2, wherein the ergoline derivative is lisuride or a salt thereof with a physiologically compatible acid.

**4.** The use according to one of the claims 1 to 3, wherein a covering layer is provided on the side of the matrix and/or active ingredient reservoir that faces away from the skin.

**5.** The use according to one of the claims 1 to 4, wherein the matrix and/or diffusion barrier comprise as their main matrix component a substance selected from the group consisting of polyacrylate, polyurethane, cellulose ether, silicone, polyvinyl compounds, silicate and mixtures of these substances as well as copolymers of these polymeric compounds, particularly polyacrylate.

**6.** The use according to one of the claims 1 to 5, wherein the diffusion barrier comprises as its main barrier component a synthetic polymer selected from the group consisting of cellulose ester, cellulose ether, silicone, polyolefin and mixtures as well as copolymers of these substances.

**7.** The use according to one of the claims 1 to 6, wherein the matrix and/or the active ingredient reservoir and/or the diffusion barrier contains a penetration-enhancing agent that is preferably selected from the group consisting of C1-C8 aliphatic, cycloaliphatic and aromatic alcohols, saturated and unsaturated C8-18 fatty alcohols, saturated and

unsaturated C8-18 fatty acids, hydrocarbons and hydrocarbon mixtures, fatty acid esters from C3-19 fatty acids and C1-6 alkyl monools, dicarboxylic acid diesters from C4-8 dicarboxylic acids and C1-6 alkyl monools, and mixtures of these substances.

8. Use of a TTS according to one of the claims 1 to 7 for the preparation of an agent for the treatment or prevention of premenstrual syndrome or its symptoms wherein the preferred F value is in the range from 0,1 to 0,5 $\mu g/cm^2/h$.

9. Use of a TTS according to one of the claims 1 to 7 for the preparation of an agent for lactation inhibition wherein the preferred F value is in the range from 0,1 to 0,5 $\mu g/cm^2/h$.

**Revendications**

1. Utilisation d'un système thérapeutique transdermique (TTS) présentant une couche de médicament qui contient au moins une matrice contenant un principe actif et/ou un réservoir de principe actif et du côté de la peau, pour ce qui est du réservoir de principe actif, une barrière de diffusion perméable au principe actif et comme principe actif, un dérivé d'ergoline selon la formule I ou un sel d'addition d'acide physiologiquement acceptable de celui-ci

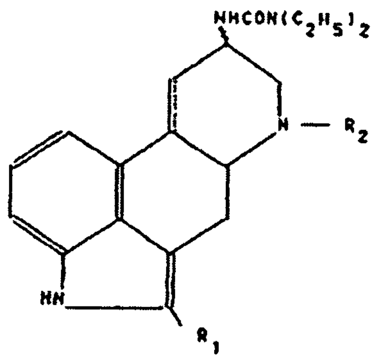

Formule I

dans laquelle ------- est une liaison simple ou une liaison double, dans laquelle $R_1$ est un atome d'hydrogène ou un atome d'halogène, en particulier, un atome de brome, et dans laquelle $R_2$ est un groupe alkyle en $C_1$ à $C_4$, pour la fabrication d'un médicament destiné au traitement du syndrome d'impatience des membres inférieurs à l'éveil *(restless legs)*.

2. Utilisation selon la revendication 1, dans laquelle la matrice et/ou la barrière de diffusion est choisie de telle manière que le flux F transdermique au travers de la peau humaine, mesuré selon l'exemple 1, soit de l'ordre de 0,1 à 2,0 $\mu g/cm^2/h$.

3. Utilisation selon la revendication 1 ou 2, dans laquelle le dérivé d'ergoline est le lisuride ou un sel d'addition d'acide physiologiquement acceptable de celui-ci.

4. Utilisation selon l'une quelconque des revendications 1 à 3, dans laquelle la matrice et/ou le réservoir de principe actif comportent sur leur face détournée de la peau une couche de revêtement.

5. Utilisation selon l'une quelconque des revendications 1 à 4, dans laquelle la matrice et/ou la barrière de diffusion comportent, comme composante principale de la matrice, une substance choisie dans le groupe formé par le polyacrylate, le polyuréthane, l'éther de cellulose, la silicone, les composés polyvinyliques, le silicate et des mélanges de ces composés et des copolymères de ces polymères, de préférence le polyacrylate.

6. Utilisation selon l'une quelconque des revendications 1 à 5, dans laquelle la barrière de diffusion comprend, en tant que composante principale, un polymère synthétique choisi dans le groupe formé par l'ester de cellulose, l'éther

de cellulose, la silicone, la polyoléfine et des mélanges et copolymères de ces substances.

**7.** Utilisation selon l'une quelconque des revendications 1 à 6, dans laquelle la matrice et/ou le réservoir de principe actif et/ou la barrière de diffusion contient un agent renforçant la pénétration, qui est choisi de préférence dans le groupe formé par les alcools aliphatiques en $C_1$ à $C_8$, cyclo-aliphatiques et aromatiques, les alcools gras en $C_8$ à $C_{18}$ saturés et insaturés, les acides gras en $C_8$ à $C_{18}$ saturés et insaturés, les hydrocarbures et les mélanges d'hydrocarbures, les esters d'acides gras issus d'acides gras en $C_3$ à $C_{19}$ et d'alkylols en $C_1$ à $C_6$ monohydriques, les diesters d'acides dicarboxyliques issus d'acides dicarboxyliques en $C_4$ à $C_8$ et d'alkylols monohydriques en $C_1$ à $C_6$ et les mélanges de ces substances.

**8.** Utilisation d'un TTS selon l'une quelconque des revendications 1 à 7, pour la fabrication d'un médicament pour le traitement ou la prévention du syndrome prémenstruel ou de ses symptômes, F étant de préférence compris entre 0,1 et 0,5 $\mu g/cm^2/h$.

**9.** Utilisation d'un TTS selon l'une quelconque des revendications 1 à 7, pour la fabrication d'un médicament pour inhiber la lactation, F étant de préférence compris entre 0,1 et 0,5 $\mu g/cm^2/h$.